# EUROPEAN PATENT APPLICATION

(11) **EP 4 670 663 A1**
(43) Date of publication of application: **31.12.2025**
(21) Application number: 24185183.1
(22) Date of filing: 28.06.2024
(51) Int. Cl.: A61B 34/20, A61B 90/00

(54) **FIDUCIAL SCREW SYSTEM**

(71) Applicant: Stryker European Operations Limited, Carrigtwohill, Co. Cork T45 HX08 (IE)
(72) Inventor: SCHÖPP, Hans, 79110 Freiburg (DE)
(74) Representative: Röthinger, Rainer

(57) **Abstract**

The present disclosure provides a fiducial screw system (100). The system (100) comprises a fiducial screw (106) having a screw shaft (108) configured to be inserted into a bone material (102) and a screw head (110) having or forming a fastening interface (126) configured to cooperate with a fastening tool for inserting the fiducial screw (106) into the bone material (102). The system further comprises an elongated tube (112) forming a guiding channel (116), wherein the elongated tube (112) has a proximal longitudinal end (118) and a distal longitudinal end (120), and wherein the distal longitudinal end (120) is shaped to be attachable or attached to the fiducial screw (106), e.g., to its head (110), and the proximal longitudinal end (118) forms a guiding opening (122) leading into the guiding channel (116).

## Description

### Technical Field

The present disclosure relates to a fiducial screw system suitable for use in Minimal Invasive Surgery (MIS), for example in a spinal region of a patient.

### Background

A fiducial marker or simply fiducial is an object that is placed in the field of view of a medical imaging system and that appears in the image produced, for use as a point of reference or measure. Fiducials are configured to be detectable when being imaged by a medical imaging system (e.g., they are made of a radiopaque material such as a metal).

It is commonly known to use fiducials for registering medical image data, such as computer tomography (CT) image data of a patient, relative to a coordinate system of a surgical tracking system that tracks a patient or a surgical tool during a surgical procedure. Based on the registration, it is possible to visualize the position of the tracked surgical tool relative to the medical image data and thus to reliably navigate the surgical tool during the surgical procedure. The registration by means of fiducials may be made initially, that is to say prior to the surgical procedure, as well as during the surgical procedure, for example in case a relative movement of the imaged bone structures such as vertebrae with respect to each other has been determined.

Fiducials can also be used for verifying navigation accurateness before or during a surgical procedure. To this end, a fiducial placed, for example, on a patient's skin is touched with a tracked navigation pointer. It is then determined whether or not the point touched by the navigation pointer corresponds to the previously determined location of the fiducial in the coordinate system of the surgical tracking system.

As said, certain types of fiducials are placed on the patient's skin. In many surgical situations, such types of fiducials result in a poor navigation accuracy due to their distance to the bony structure that is to be surgically treated. To improve navigation accuracy, other types of fiducials are configured as bone screws that are inserted into the bone material that is to be treated. A drawback of such invasive fiducials is that tissue has to be removed to expose the bone material for receiving the fiducials. Furthermore, for a verification of navigation accuracy prior to or during the surgical procedure with a navigation pointer, a relatively wide opening has to be made in the patient's tissue for allowing the surgeon to see the fiducials and to touch them with the navigation pointer. The need to remove tissue in a relatively wide region around the fiducials is particularly undesired in MIS cases in which even during the surgical procedure only a minimal amount of tissue has to be opened. While the fiducials can be placed in bone material using MIS techniques, finding and touching the fiducials, in particular deeply placed fiducials, by using a navigation pointer or similar tools may cause issues using MIS techniques.

### Summary

There is a need to overcome one or more of the above discussed and other problems in the context of invasively placed fiducials. For example, in this context there is a need for an increased navigation accuracy while minimizing the surgical impact on the patient.

According to an aspect of the present disclosure, a fiducial screw system is provided, wherein the system comprises a fiducial screw having a screw shaft configured to be inserted into a bone material and a screw head having or forming a fastening interface configured to cooperate with a fastening tool for inserting the fiducial screw into the bone material. The system further comprises an elongated tube forming a guiding channel, wherein the elongated tube has a proximal longitudinal end and a distal longitudinal end, the distal longitudinal end being shaped to be attachable or attached to the fiducial screw and the proximal longitudinal end forming a guiding opening leading into the guiding channel.

The patient's bone anatomy receiving the fiducial screw may be any bone anatomy, such as, for example, a vertebra of a spine. The fiducial screw may be a conventional bone screw adapted for the intended use (e.g., the fixation of fracted bone or of an implant), or a specific bone screw tailored for being used in combination with the elongated tube. The fiducial screw may be made of a biocompatible material.

The screw shaft may be configured with a profile adapted to fixedly attach the fiducial screw to the specific patient's bone anatomy. The screw shaft may be threaded over at least a part of its longitudinal extension. Also a length and/or a width of the screw shaft may be adapted to the specific patient's bone anatomy.

The screw head may be integrally formed with the screw shaft. The screw head may have a width dimension that is larger than a width dimension of the screw shaft. The fastening interface may be integrally formed with the screw head, for example by means of a specific groove or groove pattern provided in an upper surface of the screw head and adapted to the shape and dimension of the fastening tool, or may be part of a further component of the screw head fastened to the portion of the screw head adjoining the screw shaft.

In some variants, the fiducial screw may not be intended to be used as a surgical bone screw for fixing fractured or impaired bone parts, but may be fixed to the bone material solely for use as a point of reference or measure. In particular, prior to the actual surgical procedure, the fiducial screw system with the fiducial screw may be fixed to a bone material and may be captured by a medical imaging system (i.e. an intra-operative scan or imaging) so as to be represented by medical imaging data generated in advance of the surgical procedure, for example for at least one of image registration purposes or navigation purposes.

The elongated tube may be integrally formed or composed of one or more parts. The guiding channel of the elongated tube may have a circular cross-section. The guiding channel may be surrounded by one or more guiding walls. The guiding channel may form a hollow inner space of the elongated tube. The one or more guiding walls may provide an outer circumferential surface and an inner circumferential surface of the elongated tube. The guiding walls may provide a distal end surface of the elongated tube. The distal end surface may be provided with an opening for receiving the fiducial screw for attaching the fiducial screw to the elongated tube.

In an implementation of the aspect of the present disclosure, the elongated tube may be configured to be attached or attachable to the screw head of the fiducial screw, for example in a movable or flexible manner. As such, the elongated tube may be movable with respect to the fiducial screw, in particular with respect to the fiducial screw when being inserted into the bone material. The elongated tube may be movable when inserting a navigation pointer or a surgical tool (e.g., a surgical screw driver) through the guiding channel to the screw head. The elongated tube and the fiducial screw may be so flexibly attached that the elongated tube and the fiducial screw are not linearly displaceable or at least not substantially linearly displaceable with respect to each other in a longitudinal direction of the fiducial screw and/or of the elongated tube. The elongated tube may be configured to be attached or attachable to the screw head so that the elongated tube is not detachable from the screw head.

The elongated tube may be attached or attachable to the screw head so as to be pivotable with respect to a longitudinal axis of the fiducial screw, in particular pivotable in two or more planes comprising a longitudinal axis of the fiducial screw, in particular pivotable in all planes comprising a longitudinal axis of the fiducial screw. The elongated tube may be pivotable in only one plane comprising the longitudinal axis of the fiducial screw. The intended pivoting movement may be achieved by any kind of pivot connection, e.g., a pivoting screw-fitting, mutually intertwining connection features of or attached to the fiducial screw and the elongated tube, which allow the intended pivoting movement, and so on. The elongated tube may be pivotable over an angle range of at least 90°, any angle range lying between 90° and 45°, or any angle range lying between 45° and 0°.

The elongated tube may be attached or attachable to the screw head so as to be rotatable around a longitudinal axis of the fiducial screw. The elongated tube may be rotatable over an angle range of at least or exactly 360°, any angle range lying between 360° and 180°, or any angle range lying between 180° and 0°. The intended rotational movement may be achieved by any kind of rotation connection. The rotational movement may be provided in combination with any kind of pivoting movement/pivoting connection, in particular with any kind of pivoting movement/pivoting connection described above.

In a configuration of the present disclosure, the elongated tube and the screw head may comprise mutually intertwining connection features for providing a joint mechanism, in particular for providing a ball-and-socket joint. The joint mechanism may comprise one or more joint components. In case that more than one joint components are provided, the joint mechanism may be so configured that the more than one joint components pivot, tilt and/or rotate with respect to the same point and/or axis. The joint mechanism may provide any kind and number of degrees of freedom. The joint mechanism may be or comprise a rotational joint mechanism. In one configuration, the joint mechanism, in particular the ball-and-socket joint, may be provided by a separate component or components directly attached to the elongated tube and/or screw head which comprise mutually intertwining connection features for providing the joint mechanism, in particular for providing the ball-and-socket joint.

A distal longitudinal end of the elongated tube may have an increased width dimension (e.g., compared to a proximal longitudinal end of the elongated tube). A distal longitudinal end portion comprising the distal longitudinal end may have an increased width dimension. The increased width dimension may at least partly form a connection feature for connecting the elongated tube and the fiducial screw (e.g., its screw head), in particular a connection feature of a joint mechanism. The distal longitudinal end and/or end portion may be integrally formed with the adjoining, more proximal portion of the elongated tube or may be provided by a separate component, which may be fixedly connected to the distal longitudinal end and/or end portion.

The elongated tube may be rigidly configured or flexibly configured. A rigid configuration may be provided by means of the material or materials of the elongated tube (e.g., a metallic material) and/or by means of the specific configuration (e.g., wall thickness or cross-sectional shape of the elongated tube). A "rigid" configuration may be realized by an elongated tube that does not deform or substantially does not deform when inserting a tool into the tube. Analogously, a flexible configuration may be provided by means of the material or materials of the elongated tube (e.g., a rubber material) and/or by means of the specific configuration (e.g., wall thickness or cross-sectional shape of the elongated tube). A "flexible" configuration may be realized by an elongated tube that can deform when inserting a tool. For example, a bent shape of the elongated tube may straighten when inserting the tool.

In some implementations, the elongated tube may be directly attached or attachable to the screw head. The elongated tube may be directly attached or attachable via one or more connection features provided at the elongated tube and the fiducial screw, respectively. The connection features may be integrally formed with the elongated tube and/or the screw. The connection features of the screw and the elongated tube may have mutually corresponding shapes. In other implementations, the elongated tube and the screw are connected via one or more components connected to the elongated tube and/or the screw, respectively.

The elongated tube may be a circumferentially closed tube. The elongated tube may be circumferentially closed without any gaps or slots thereby forming a continuous guiding wall. The elongated tube may remain circumferentially closed, in particular throughout the overall surgical procedure.

The elongated tube may comprise a radiolucent material. In addition or as an alternative, the screw head (and, optionally, the entire fiducial screw) may comprise a radio-opaque material. The radiolucent material may be at least radiolucent for the electromagnetic radiation of a medical imaging apparatus configured to generate medical images of the patient's bone anatomy (e.g., a CT-based imaging apparatus). Radiolucent material may comprise material that is substantially transparent for the radiation in a specific energy region, in particular for the radiation used in the medical imaging apparatus. Substantially transparent may mean transparent for more than 95% of the incoming radiation, for more than 90% of the incoming radiation, for more than 85% of the incoming radiation or for more than 80% of the incoming radiation. The radio-opaque material may be at least radio-opaque for the electromagnetic radiation of a medical imaging apparatus configured to generate medical images of the patient's bone anatomy. Radio-opaque material may comprise material that is substantially opaque (intransparent) for the radiation in a specific energy region, in particular for the radiation used in the medical imaging apparatus. Substantially opaque may mean opaque for more than 95% of the incoming radiation, for more than 90% of the incoming radiation, for more than 85% of the incoming radiation or for more than 80% of the incoming radiation.

In some variants, the screw head may be configured to protrude into a distal portion of the guiding channel in a state where the elongated tube is attached to the fiducial screw. The connection between the screw and the elongated tube may be configured such that the screw head protrudes into a distal portion of the guiding channel in a state where the elongated tube is attached to the fiducial screw. In these variants, the guiding channel may guide any tool (e.g., a navigation pointer) inserted into the guiding channel from the guiding opening to the screw head. The screw head may be surrounded by the guiding channel so as to not come into contact with the surrounding tissue.

The elongated tube may have a length dimension sized to allow the elongated tube to project beyond a patient's outer skin surface when the fiducial screw is inserted into the bone material. The elongated tube sized to project beyond a patient's outer skin surface may be visible and accessible from outside the patient's outer skin surface without the need to provide a wide opening in the tissue. A typical range for the length dimension of the elongated tube is from 5 cm to 15 cm, for example between 6 cm and 8 cm, between 9 cm and 11 cm, or between 12 and 14 cm.

The guiding channel may have a width dimension sized to allow a distal end of the fastening tool to be moved from the guiding opening through the guiding channel to the fastening interface of the screw head. The width dimension may be perpendicular to the longitudinal direction of the elongated tube. The width dimension may be sized to be slightly larger than a corresponding, largest width dimension of the distal end of the fastening tool. A typical range for the width dimension of the guiding channel is from 4 mm to 8 mm, for example between 4 mm and 5 mm, between 5 mm and 6 mm, between 6 mm and 7 mm or between 7mm and 8 mm.

The guiding channel may have a length dimension sized to allow a proximal end portion of the fastening tool to protrude out from the guiding channel. The proximal end portion may comprise a grip portion configured for gripping the fastening tool.

The guiding channel may have a width dimension sized to allow a distal end of a navigation pointer to be moved from the guiding opening through the guiding channel to the screw head. The width dimension may be perpendicular to the longitudinal direction of the elongated tube. The width dimension may be sized to be slightly larger than a corresponding, largest width dimension of the distal end of the navigation pointer. The navigation pointer may carry one or more markers trackable by a surgical tracking system and located outside the elongated tube in a state where the navigation pointer contacts the screw head. The guiding channel may have a length dimension sized to allow a proximal end portion of the navigation pointer to protrude out of the guiding channel. The proximal end portion may comprise a grip portion configured for gripping the navigation pointer.

In variants having a guiding channel with a width dimension sized to allow a distal end of a navigation pointer to be moved from the guiding opening through the guiding channel to the screw head, the fiducial screw system may comprise at least one tool configured to be inserted into the guiding channel, such as at least one of the fastening tool and the navigation pointer. The fastening tool or the navigation pointer may be a trackable tool provided with one or more (e.g., three or more) trackable optical markers. The one or more markers may be passive (light-reflective) or active (light-emitting) markers. The one or more markers may be provided on or attached to a grip portion of the fastening tool or the navigation pointer. The one or more markers may be configured as spheres. At least some of the one or more markers may be provided with a distance from the longitudinal axis of the navigation pointer. The one or more markers may be reflective with respect to, or emit light in, an energy range in which an optical system is sensitive (e.g., in an infrared spectrum).

According to another aspect, a surgical navigation system may comprise the fiducial screw system with at least one of the fastening tool and the navigation pointer. The surgical navigation system may further comprise an optical sensor capable of detecting light reflected or emitted by the one or more markers and of generating a first sensor signal indicative of the detected light. The optical sensor may be integrated in a camera or camera system (e.g., in a stereo camera).

The camera or camera system may be part of a surgical tracking system comprised by the surgical navigation system and having its associated tracking coordinate system. The position of the fastening tool or the navigation pointer in the tracking coordinate system may be detected by or using the optical sensor. Upon the navigation pointer pointing at a dedicated point, the position of the dedicated point may be detected by the surgical navigation system. In particular, upon the navigation pointer contacting the fiducial screw head, the position of the fiducial screw head may be detected and, optionally, verified (e.g., relative to previously determined position in the tracking coordinate system).

Tracking of at least one of the fastening tool and the navigation pointer may be achieved by means of a navigation controller capable of receiving the first sensor signal and of tracking at least one of the fastening tool and the navigation pointer based on the received first sensor signal. In particular, upon the tracked navigation pointer contacting the fiducial screw head, the position of a tip of the navigation pointer and, thus, of the fiducial screw head may be determined (e.g., in the tracking coordinate system).

In an implementation, the surgical navigation system may comprise a medical imaging system configured to generate medical imaging data of the patient's bone anatomy with the fiducial screw being inserted therein. In this or another implementation, the surgical navigation system may comprise the medical imaging data (e.g., stored on a hard drive or semiconductor memory). The navigation controller may be configured to register in a coordinate system (e.g., the tracking coordinate system) the medical imaging data of the patient's bone anatomy, with the fiducial screw inserted therein, based at least on the first sensor signal data.

In a further implementation, the navigation controller may be configured to verify whether the medical imaging data of the patient's bone anatomy, with the fiducial screw inserted therein, are correctly registered in a coordinate system (e.g., the tracking coordinate system) based at least on second sensor signal data by the optical sensor based on a second signal received from the optical sensor (e.g., the optical markers of the navigation pointer). In particular, it may thus be verified that an initial registration is still valid.

The fiducial screw system according to the present disclosure is particularly suitable to be used in MIS surgery. In particular, the surgical navigation system may be a MIS-compliant surgical navigation system.

### Brief Description of the Drawings

Further details, advantages and aspects of the present disclosure will become apparent from the following embodiments taken in conjunction with the drawings, wherein:
- Figure 1: schematically shows a fiducial screw system according to one aspect of the present disclosure, in a state where a fiducial screw is inserted into a bone material and an elongated tube protrudes beyond an outer skin surface of a patient;
- Figure 2: schematically shows a fiducial screw system according to another aspect of the present disclosure, in a state where a fiducial screw and am elongated tube are attached to each other; and
- Figure 3: schematically shows a surgical navigation system with the fiducial screw system of Figure 2 and a navigation pointer.

### Detailed Description of the Drawings

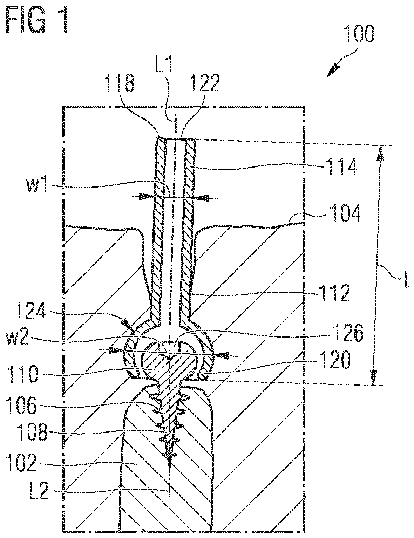

Figure 1 schematically shows an embodiment of a fiducial screw system 100 in a state where it is attached to a bone material 102 of a patient and protrudes from a skin surface 104 of the patient. The fiducial screw system 100 comprises a fiducial screw 106 having a screw head 108 and a screw shaft 110. In the shown embodiment, the screw shaft 110 is threaded and extends along a longitudinal axis L1 of the fiducial screw 106 in a longitudinal direction.

The fiducial screw system 100 further comprises an elongated tube 112 having a guiding wall 114 surrounding a guiding channel 116 defining a hollow inner space of the elongated tube 112. In particular, the guiding wall 114 completely surrounds the guiding channel 116 so that the elongated tube 112 is a circumferentially closed tube.

The elongated tube 112 has a proximal longitudinal end 118 and a distal longitudinal end 120. The guiding channel 116 has an open end both at the proximal longitudinal end 118 and the distal longitudinal end 120 of the elongated tube 112. The proximal longitudinal open-ended end 118 forms a guiding opening 122.

The elongated tube 112 has a length dimension /along a longitudinal axis L2 of the elongated tube 112, which extends in a longitudinal direction, that is greater than a width dimension *w1, w2* in a width direction being perpendicular to the longitudinal direction. In particular, at its distal longitudinal end 120, the elongated tube 112 has a width dimension w2 that is greater than the width dimension w1 at its proximal longitudinal end 118 and in an intermediate region between the proximal longitudinal end 118 and the distal longitudinal end 120. The distal longitudinal end 120 is shaped to be attachable to the fiducial screw 106 and in particular to the screw head 110 of the fiducial screw 106.

As becomes apparent from Figure 1, the elongated tube 112 widens in a distal end portion 124 so that the guiding wall 114 has a shape that is adapted to the shape of the screw head 110 for allowing the elongated tube 112 to be (e.g. detachably) attached to the fiducial screw 106, in particular, to be moveably attached to the fiducial screw 106. Specifically, the widened distal end portion 124 of the elongated tube 112 and the screw head 110 are configured to form mutually intertwining connection features for being directly attached to each other. In the present embodiment, the distal longitudinal end portion 124 and the screw head 110 form a ball-and-socket joint. The ball-and-socket joint allows the elongated tube 112, when being attached to the fiducial screw head 110 and the fiducial screw 106 being inserted into the bone material 102, to be pivoted in all planes comprising the longitudinal axis L2 of the fiducial screw 106 and, optionally, to be rotatable around the longitudinal axis L2 of the fiducial screw 106.

The open-ended proximal longitudinal end 118 of the elongated tube 112 forming the guiding opening 122 is configured to provide an access for a fastening tool (e.g., a surgical screw driver) and/or for one or more other surgical tools. The guiding channel 116 is configured to receive at least a respective distal portion of tool which has entered the guiding channel 116 via the guiding opening 122. In particular, the guiding channel 116 has a width dimension *w1,* w2and a length dimension /allowing at least a distal portion of the fastening tool and/or the one or more surgical tools to be moved from the guiding opening 122 through the guiding channel 116 to the screw head 110. The distal tip of the fastening tool is configured to cooperate with a fastening interface 126 of the screw head 110 for fastening the fiducial screw 106 in the bone material 102. For example, the fastening tool may be a screwdriver, and the elongated tube 112 has a width dimension *w1, w2* and a length dimension / which allows the screwdriver blade to have access to the guiding channel 116 via the guiding opening 122 and to be moved from the guiding 122 opening through the guiding channel 116 to the fastening interface 126 of the screw head 110.

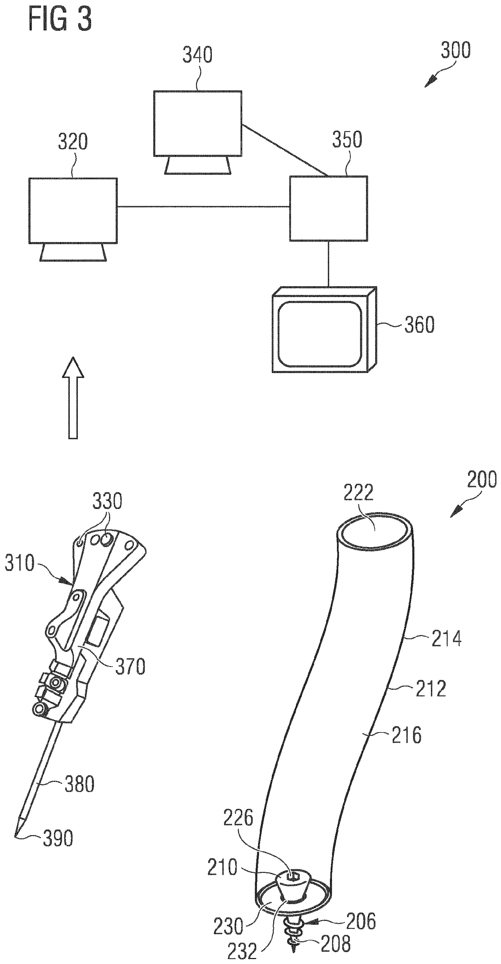

In a preferred variant, each one of the one or more surgical tools to be used with the fiducial screw system 100 comprises one or more optical markers to allow determining a position of the respective surgical tool. In a further preferred variant, the one or more surgical tools comprise a navigation pointer. An example of a navigation pointer is shown in Figure 3 and shall be described in detail further below.

Returning to Figure 1, the elongated tube 112 is made of a rigid material. Furthermore, the elongated tube 112 comprises a radiolucent material. As an example, the elongated tube 112 made be made from stainless steel or titanium.

In the state shown in Figure 1 where the fiducial screw system 100 is attached to a bone material 102 of a patient, the elongated tube 112 protrudes beyond the patient's skin surface 104. Or, in other words, the elongated tube 112 is long enough to be able to protrude beyond the skin surface 104 so that the proximal longitudinal end 118 is no longer covered by the skin or other tissue, and so that the guiding opening 122 is accessible by the navigation pointer or other surgical tool.

The fiducial screw 106 is inserted with its threaded screw head 110 into the patient's bone material 102, thereby being fixedly attached. The elongated tube 112 is movably attached to the fiducial screw head 110 at its distal end portion 124 by means of the above-described ball-and-socket joint formed by the mutually intertwining screw head 110 and widened distal end portion 124 of the elongated tube 112. In particular, the fiducial screw head 110 protrudes into the distal end portion 124 of the guiding channel 116 and is surrounded by the distal end portion 124 of the guiding wall 114 having a shape adapted to the shape of the fiducial screw head 110.

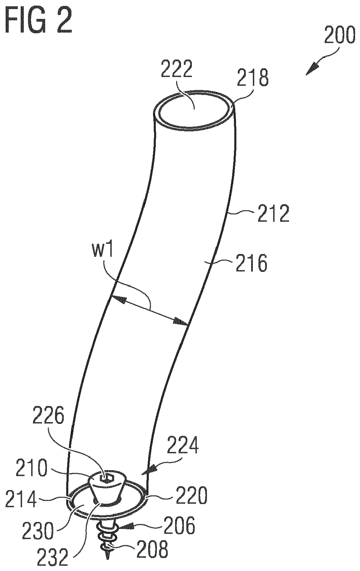

Figure 2 of the drawings shows another embodiment of a fiducial screw system 200 according to the present disclosure. The features of the embodiment of Figure 2 which correspond to respective features of the embodiment of Figure 1 are designated with the same reference numerals increased by 100. Furthermore, the explanations made with respect to the embodiment of Figure 1 also apply to the embodiment of Figure 2, unless otherwise stated in the following when describing certain differences.

In particular, as is seen in Figure 2, also the fiducial screw system 200 also comprises an elongated tube 212 and a fiducial screw 206 which are movably attachable or attached (as shown in Fig. 2) to each other. However, deviating from the elongated tube 112 of Figure 1, the distal end portion 224 of the elongated tube 212 does not have an increased width dimension. Instead, the elongated tube 212 has a substantially same width dimension w1 along its longitudinal extension, i.e. from the proximal longitudinal end 218 to the distal longitudinal end 220. In particular, the distal end portion 224 and the screw head 210 are not shaped to form a joint mechanism such as a ball-and-socket joint. Furthermore, the elongated tube 212 is flexibly configured, and not rigidly configured as in the embodiment of Fig. 1. More particularly, the elongated tube 212 is made of a flexible material or comprises a flexible material (e.g., a bio-compatible rubber material).

The guiding channel 216 is not open-ended at its distal longitudinal end 220, but is closed by a correspondingly shaped collar or lid 230 or end surface formed by the guiding wall 214, which comprises an opening 232 sized for receiving the screw shaft 208 of the fiducial screw 206 for flexibly attaching the elongated tube 212 to the fiducial screw 206. As is seen in Figure 2, the fiducial screw shaft 208 is received by and protrudes through the opening 232 so that a major part of the screw shaft 208 is located outside the elongated tube 212. The screw head 210, which has a larger width dimension than the screw shaft 208, protrudes into the distal end portion 224 of the guiding channel 216 and prevents the fiducial screw 206 from slipping through the opening.

Thus, in both embodiments, the elongated tube 112, 212 is movably attached or may be movably attached to the fiducial screw 206. In Figure 1, the elongated tube 112 is rigidly configured and the movability is achieved by means of the ball-and-socket joint, and in Figure 2, the movability is achieved by means of the elongated tube 212 being flexibly configured.

For attaching any of the fiducial screw systems 100, 200 of Figure 1 or Figure 2 to a patient's bone for an MIS procedure, a small stab incision is made into the patient's skin and tissue close to a patient's bone material intended for attaching the fiducial screw system 100, 200. After having made the small stab incision and thereby provided a minimally invasive access to the patient' s bone, the fiducial screw system 100, 200 with the elongated tube 112, 212 attached to the screw head 110, 210 is inserted through the incision until it reaches the bone. To this end, a tool having a rigid shaft, such as a surgical screwdriver or other fastening tool, can be used, with the shaft being inserted into the elongated tube 112, 212 to move the fiducial screw systems 100, 200 towards the target bone. In some variants, a hole for receiving the fiducial screw 106, 206 has been pre-drilled in the target bone.

If a tool different from a fastening tool has been used to move the fiducial screw system 100, 200 towards the target bone, this tool is removed again. Since the elongated tube 112, 212 protrudes beyond the outer skin surface 104 with its distal longitudinal end 120, 220 in a state where the distal tip of the fiducial screw 106, 206 contacts the bone material of the target bone, the distal tip of a fastening tool such as a screwdriver blade, may then be inserted into the guiding channel 116, 216 and along the guiding channel 116, 216 until it reaches a correspondingly shaped fastening interface 126, 226 at the fiducial screw head 110, 210. The distal tip is then brought into engagement with the fastening interface 110, 210. Afterwards, the fiducial screw 106, 206, with the elongated tube 112, 212 attached thereto, is screwed into the bone material 102. After having fixedly attached the fiducial screw 106, 206 to the bone, the distal tip of the fastening tool is removed from and out of the guiding channel 116, 216.

The guiding channel 116, 216 is configured to guide the fastening tool safely and reliably to the fastening interface 126, 226 for attaching the fiducial screw system 100, 200 to the target bone. It further provides for smooth and seamless insertion of the fastening tool, thereby protecting the surround tissue against potential injuries.

Furthermore, due to the movable (e.g., pivotable) attachment, the fastening tool is safely guided without providing a lever arm that could move the fiducial screw 106, 206 before being fixedly attached or hurt the surrounding tissue. In particular, in the embodiment of Figure 1, the ball-and-socket joint allows a pivoting of the elongated tube 112 in all planes comprising the longitudinal axis L2 of the fiducial screw and, optionally, a rotation around the longitudinal axis L2 and, thus, avoids providing a lever arm. Moreover, in the embodiment of Figure 2, the flexibly configured elongated tube 212 avoids providing a lever arm while being sufficiently sturdy to allow the distal tip of the fastening tool to enter the guiding channel 216 and reach the fiducial screw head 210 in a guided manner. The flexibly configured elongated tube 212 may straighten during insertion of the distal tip of the fastening tool.

During a surgical procedure, a distal portion of a surgical tool trackable by a tracking system, for example a navigation pointer, may be inserted into the guiding opening 122, 222 and moved from the guiding opening 122, 222 and through the guiding channel 116, 216 until the distal tip of the surgical tool contacts the fiducial screw head 110, 210. In that way, since the position of the distal tip in the surgical navigation system can be determined, also the position of the screw head 110, 210 of the fiducial screw system 100, 200 is determined.

Thus, the guiding channel 116, 216 is also configured to guide the navigation pointer safely and reliably to the fiducial screw head 110, 210. It further also provides for smooth and seamless tool insertion, thereby protecting the surrounding tissue against potential injuries, as explained above with reference to the fastening tool.

Figure 3 shows a surgical navigation system 300 which comprises the fiducial screw system 200, a trackable surgical tool 310 and an optical sensor 320. In Figure 3, the surgical navigation system 300 is shown with the fiducial screw system 200 of Figure 2. However, the surgical navigation system 300 can be used in connection with any of the fiducial screw system embodiments encompassed by the present disclosure. Furthermore, in Figure 3, the trackable surgical tool 310 is a navigation pointer comprising a grip portion 370 with a plurality of passive markers 330 and a tip portion 380 with a distal pointing tip 390. Of course, the present disclosure can also be implemented with any other surgical tools having a portion that needs to be guided towards the head of a fiducial screw 106, 206

It is noted that Figure 3 is only a schematic drawing, which does not reproduce the correct relative dimensions between the individual components of the surgical navigation system 300. For example, the tip portion 380 is depicted too short relative to the length of the elongated tube 212. As is described above, when the tip portion 380 is guided through the guiding opening 222 to the fiducial screw 206, the tip portion 380 is long enough to contact the fiducial screw head 210 with its distal tip 390, while the grip portion 370 is located outside the elongated tube 212.

The optical sensor 320 can be comprised by a camera of a surgical tracking system, in particular a stereo camera. The surgical tracking system may be configured to track the position and/or orientation of trackers with optical markers in a tracking coordinate system (e.g., having its origin in a center of the optical sensor 320). Such trackers may be integrated in or attached to a patient, the surgical tool 310, a medical imaging device (not shown), and so on. The surgical tracking system may be integrated (e.g., as a software routine) into the surgical navigation system 300.

The optical sensor 320 illustrated in Fig. 3 is configured to detect a light spectrum that is reflected (in the case that the tracker comprises one or more passive markers) or emitted (in the case that the tracker comprises one or more active markers) by the one or more markers 330 of the trackable surgical tool 310. The optical sensor 320 may be for example configured to detect light in the IR spectrum. In such a case, the surgical navigation system 300 is less affected by ambient light. The optical sensor 320 is configured to generate sensor signals indicative of the detected light. Different sensor signals may be generated for different trackers.

The surgical navigation system 300 further comprises a medical imaging system 340. The medical imaging system 340 may comprise, for example, a CT system or a magnetic resonance imaging (MRI) system. The medical imaging system 340 is configured to generate imaging data of a patient's bone anatomy with the fiducial screw system 100, 200 attached to the patient's bone anatomy.

The surgical navigation system 300 further comprises a navigation controller 350 that is configured to generate, from the sensors signals output by the optical sensor 320, sensor data and to register or track the localizable surgical tool 310 based on the sensor data. The navigation controller 350 may be part of the camera or camera system comprising the optical sensor 320. The navigation controller 350 may integrate algorithms of the tracking system. In some variants, the navigation controller 350 may be a separate device that is not part of the surgical navigation system 300, such as a computer that manages patient data, but that can additionally be used to compute the sensor data of the optical sensor 320.

The surgical navigation system 300 may further comprise or be connected to an output device 360. The output device 360 comprises at least one of a display, a speaker, a beamer, and a haptic feedback device. In Figure 3, the output device 360 comprises a display. The output device 360 is configured to output information and/or instructions that result from tracking the localizable surgical tool 310 (e.g., navigation information and/or navigation instructions).

The output information and/or instructions may comprise at least one of a visual display of the tracked surgical tool 310, a visual display of the tracked surgical tool 310 relative to patient data acquired by the imaging device 340, and an acoustic signal indicative of a position of the tracked surgical tool 310 (e.g., relative to a virtual boundary with respect to a patient). The instructions may comprise at least one of a visual representation of a target shape, target position and target orientation of the tracked surgical tool 310, and a voice output guiding the surgeon.

In an aspect of the present disclosure, medical imaging data of a patient's bone anatomy with a fiducial screw system 100, 200 according to the present disclosure attached thereto is generated by means of the medical imaging device 340, for example a CT system. Since the elongated tube 112, 212 may comprise a radiolucent material and the screw head 110, 210 (or the entire fiducial screw 106, 206) may comprise a radio-opaque material, the medical imaging data will be representative of the patient's bone anatomy and the screw head 110, 210 (or the entire fiducial screw 106, 206). The elongated tube 112, 212, on the other hand, may not generate any artefacts in these imaging data, which can be desirable for a high navigation accuracy.

During a surgical procedure, the medical imaging data thus obtained may be used to display one or more images of the patient's bone anatomy on the visual display of the output device 360. The displayed patient's bone anatomy may be overlaid by a visual display of a tracked surgical tool (or at least a visual display representative of the position thereof) in order to assist the surgeon to navigate the surgical tool relative to the surgical imaging data. This surgical tool may be different from the navigation pointer 310. It may, for example, be a surgical drill, burr or saw.

A prerequisite for a precise and reliable navigation during the surgical procedure is that the medical imaging data and the optical sensor data generated during the surgical procedure, based on which the visual display of the tracked surgical tool or a representation of its position is generated, are correlated with each other. That is to say, the surgical system has to "know" which point in the medical image corresponds to which point in a tracking coordinate system in which the optical sensor 320 tracks the surgical drill or other surgical tool. The correlation process is called a registration.

The fiducial screw system 100, 200 according to the present disclosure may be used for registering the medical imaging data relative to the tracking coordinate system. For that purpose, a navigation pointer, such as the navigation pointer 310 shown in Figure 3 of the drawings, may be introduced into the guiding opening 122, 222 of the elongated tube 112, 212 and moved through the guiding channel 116, 216 to the fiducial screw head 110, 210 until it contacts the fiducial screw head 110, 210 with its distal tip 390. A position of the distal tip 390 of the navigation pointer 310 contacting the fiducial screw head 112, 212, which substantially corresponds to the position of the fiducial screw head 112, 212 included in the first medical imaging data, is then detected and determined by means of the optical sensor 320 and the navigation controller 350 in the tracking coordinate system. For example, the navigation pointer 310 may comprise a component configured to communicate with the navigation controller 350 to signal that the pointing tip 390 currently contacts the fiducial screw head 112, 212 and that a "snap shot" of the position of the pointing tip 390, and thus of the fiducial screw head 112, 212, in the tracking coordinate system is to be taken. This procedure is typically repeated for two, three or more fiducial screw systems 100, 200 placed in the patient's bone material.

In that way, the medical imaging data and the optical sensor data may be registered, as the fiducial screw heads 112, 212 in the medical image data may be determined in a first step (e.g., automatically or semi-automatically). In a second step, the medical imaging data are placed such in the tracking coordinate system that the fiducial screw heads 112, 212 thus determined are aligned with their respective position acquired using the navigation pointer 310. After a successful registration, a visual display of a tracked surgical burr or other surgical tool 310, overlaid over the displayed medical imaging data in the tracking coordinate system, can be output as navigation information to a surgeon.

The navigation pointer 310 may also be used to repeatedly verify the correct registration of the medical imaging data in the course of the surgical procedure (also in scenarios in which the registration has been performed without using the navigation pointer 310). Known error sources for an incorrect navigation is an incorrect registration which might occur during the surgical procedure, e.g. caused by a displacement of the patient's bone anatomy. For verifying that the original registration is still valid, a surgeon may, regularly and/or on suspicion of a misregistration, contact the fiducial screw head 106, 206 with the tip 390 of the navigation pointer 310 by moving it through the guiding channel 116, 216 of the elongated tube 112, 212 and confirm that the displayed position of the tip 390 of the surgical tool 310 coincides with the known/suspected position of the fiducial screw head 106, 206 in the tracking coordinate system.

As has become apparent from the above embodiments, for placing the fiducial screw system according to the present disclosure in or at a patient's bone anatomy, only a small stab incision has to be made. Thus, there is no need to make a wide opening in the tissue for placing the fiducial bone screw system.

Furthermore, in a state where the fiducial screw system is fixed to a patient's bone anatomy, the elongated tube may protrude beyond a patient's outer skin surface so as to be visible for the surgeon and allow easy access to the fiducial screw although the fiducial screw is covered by tissue. Thus, also for accessing the fiducial screw, it is not necessary to make a wide opening in the tissue, in accordance with the paradigm of MIS. With the fiducial screw system according to the present disclosure comprising the elongated tube, the fiducials can always be found back easily by means of a navigation pointer or other surgical tool (e.g., fastening device for removing the fiducial screw system after the surgical procedure). In particular, a surgeon/user does not need to see the fiducial screw in order to be able to touch it with a navigation pointer or find it with a fastening device.

Furthermore, when inserting the surgical tool, e.g. the navigation pointer, into the elongated tube and moving it towards the fiducial screw head, the guiding channel allows for a smooth and seamless insertion of the surgical tool tip portion. It guides the tip portion safely to the fiducial screw head and protects the surrounding tissue against injuries.

The fiducial screw system may be used to reduces the invasiveness of fiducial screws, and thus is particularly advantageous for MIS.

## Claims

1. A fiducial screw system (100, 200), the system (100, 200) comprising
a fiducial screw (106, 206) having a screw shaft (108, 208) configured to be inserted into a bone material (102) and a screw head (110, 210) having or forming a fastening interface (126, 226) configured to cooperate with a fastening tool for inserting the fiducial screw (106, 206) into the bone material (102); and
an elongated tube (112, 212) forming a guiding channel (116, 216), wherein the elongated tube (112, 212) has a proximal longitudinal end (118, 218) and a distal longitudinal end (120, 220), the distal longitudinal end (120, 220) being shaped to be attachable or attached to the fiducial screw (106, 206) and the proximal longitudinal end (118, 218) forming a guiding opening (122, 222) leading into the guiding channel (116, 216).

2. The system (100, 200) of claim 1, wherein
the elongated tube (112, 212) is configured to be attached or attachable to the screw head.

3. The system (100, 200) of claim 1 or 2, wherein
the elongated tube (112, 212) is attached or attachable to the fiducial screw (106, 206) so as to be pivotable with respect to a longitudinal axis (L1) of the fiducial screw (106, 206), in particular pivotable in two or more planes comprising a longitudinal axis (L1) of the fiducial screw (106, 206), in particular pivotable in all planes comprising a longitudinal axis (L1) of the fiducial screw (106, 206).

4. The system (100, 200) of any of the preceding claims, wherein
the elongated tube (112, 212) is attached or attachable to the fiducial screw (106, 206) so as to be rotatable around a longitudinal axis (L1) of the fiducial screw (106, 206).

5. The system (100) of any of the preceding claims, wherein
the elongated tube (112) and the fiducial screw (106, 206), in particular the screw head (110), comprise mutually intertwining connection features for providing a joint mechanism, in particular for providing a ball-and-socket joint.

6. The system (100) of any of the preceding claims, wherein
a distal end portion (124) of the elongated tube (112) has an increased width dimension.

7. The system (100) of any of claims 1 to 6, wherein
the elongated tube (112) is rigidly configured.

8. The system (200) of any of claims 1 to 4, wherein
the elongated tube (212) is flexibly configured.

9. The system (100, 200) of any of the preceding claims, wherein
the elongated tube (112, 212) is directly attached or attachable to the screw head (110, 210).

10. The system (100, 200) of any of the preceding claims, wherein
the elongated tube (112, 212) is a circumferentially closed tube.

11. The system (100, 200) of any of the preceding claims, wherein
the elongated tube (112, 212) comprises a radiolucent material and/or the screw head comprises a radio-opaque material.

12. The system (100, 200) of any of the preceding claims, wherein
the screw head (110, 210) is configured to protrude into a distal end portion (124, 224) of the guiding channel (116, 216) in a state where the elongated tube (112, 212) is attached to the fiducial screw (106, 206).

13. The system (100, 200) of any of the preceding claims, wherein
the elongated tube (112, 212) has a length dimension (I) sized to allow the elongated tube (112, 212) to project beyond a patient's outer skin surface (104) when the fiducial screw (106, 206) is inserted into the bone material (102).

14. The system (100, 200) of any of the preceding claims, wherein
the guiding channel (116, 216) has a width dimension (w1, w2) sized to allow a distal end portion of the fastening tool to be moved from the guiding opening (122, 222) through the guiding channel (116, 216) to the fastening interface (126, 226) of the screw head (110, 210).

15. The system (100, 200) of any of the preceding claims, wherein
the guiding channel (116, 216) has a width dimension (w1, w2) sized to allow a distal end portion (380) of a navigation pointer (310) comprising one or more markers (330) to be moved from the guiding opening (122, 222) through the guiding channel (116, 216) to the screw head (110, 210).

16. The system (100, 200) of at least one of claim 14 and 15, further comprising at least one of the fastening tool and the navigation pointer (310).

17. A surgical navigation system (300), comprising
the system (100, 200) of claim 16, wherein at least one of the fastening tool and the navigation pointer (310) are provided with one or more trackable optical markers; and
an optical sensor (320) capable of detecting light reflected or emitted by the one or more optical markers (330), wherein the surgical navigation system is configured to generate a first sensor signal indicative of the detected light.

18. The surgical navigation system (300) of claim 17, further comprising
a navigation controller (350) capable of receiving the first sensor signal, generating first sensor signal data based on the received first sensor signal and tracking at least one of the fastening tool and the navigation pointer (310) based on the generated first sensor signal data.

19. The surgical navigation system (300) of claim 17 or 18, further comprising at least one of
a medical imaging system (340) configured to generate medical imaging data of the patient's bone anatomy with the fiducial screw (106, 206) being inserted therein, and the medical imaging data.

20. The surgical navigation system (300) of any of claims 17 to 19, wherein
the navigation controller (350) is configured to register in a coordinate system the medical imaging data of the patient's bone anatomy, with the fiducial screw (106, 206) inserted therein, based at least on the first sensor signal data.

21. The surgical navigation system (300) of claim 19 or 20, wherein
the navigation controller (350) is configured to verify whether the medical imaging data of the patient's bone anatomy, with the fiducial screw inserted therein, are correctly registered in a, or the, coordinate system based at least on second sensor signal data generated based on a second sensor signal received from the optical sensor (320).

## Amended claims

### Amended claims in accordance with Rule 137(2) EPC.

1. A fiducial screw system (100), the system (100) comprising
a fiducial screw (106) having a screw shaft (108) extending in a screw longitudinal direction and configured to be inserted into a bone material (102) and a screw head (110) having or forming a fastening interface (126) configured to cooperate with a fastening tool for inserting the fiducial screw (106) into the bone material (102); and
an elongated tube (112) forming a guiding channel (116), wherein the elongated tube (112) extends in a tube longitudinal direction from a proximal longitudinal end (118) to a distal longitudinal end (120), the distal longitudinal end (120) being shaped to be attachable or attached to the screw head (110) of the fiducial screw (106) so as the elongated tube (112) being pivotable with respect to a longitudinal axis (L1) of the fiducial screw (106) and the proximal longitudinal end (118) forming a guiding opening (122) leading into the guiding channel (116) and to the fastening interface (126).

2. The system (100) of claim 1, wherein
the elongated tube (112) is attached or attachable to the fiducial screw (106) so as to be pivotable in two or more planes comprising a longitudinal axis (L1) of the fiducial screw (106), in particular pivotable in all planes comprising a longitudinal axis (L1) of the fiducial screw (106).

3. The system (100) of any of the preceding claims, wherein
the elongated tube (112) is attached or attachable to the fiducial screw (106) so as to be rotatable around a longitudinal axis (L1) of the fiducial screw (106).

4. The system (100) of any of the preceding claims, wherein
the elongated tube (112) and the screw head (110) of the fiducial screw (106) comprise mutually intertwining connection features for providing a joint mechanism, in particular for providing a ball-and-socket joint.

5. The system (100) of any of the preceding claims, wherein
a distal end portion (124) of the elongated tube (112) has an increased width dimension.

6. The system (100) of any of claims 1 to 6, wherein
the elongated tube (112) is rigidly configured.

7. The system (100) of any of the preceding claims, wherein
the elongated tube (112) is directly attached or attachable to the screw head (110).

8. The system (100) of any of the preceding claims, wherein
the elongated tube (112) is a circumferentially closed tube.

9. The system (100) of any of the preceding claims, wherein
the elongated tube (112) comprises a radiolucent material and/or the screw head comprises a radio-opaque material.

10. The system (100) of any of the preceding claims, wherein
the screw head (110) is configured to protrude into a distal end portion (124) of the guiding channel (116) in a state where the elongated tube (112) is attached to the fiducial screw (106).

11. The system (100) of any of the preceding claims, wherein
the elongated tube (112) has a length dimension (l) sized to allow the elongated tube (112) to project beyond a patient's outer skin surface (104) when the fiducial screw (106) is inserted into the bone material (102).

12. The system (100) of any of the preceding claims, wherein
the guiding channel (116) has a width dimension (w1, w2) sized to allow a distal end portion of the fastening tool to be moved from the guiding opening (122) through the guiding channel (116) to the fastening interface (126) of the screw head (110).

13. The system (100) of any of the preceding claims, wherein
the guiding channel (116) has a width dimension (w1, w2) sized to allow a distal end portion (380) of a navigation pointer (310) comprising one or more markers (330) to be moved from the guiding opening (122) through the guiding channel (116) to the screw head (110).

14. The system (100) of at least one of claim 12 and 13, further comprising
at least one of the fastening tool and the navigation pointer (310).

15. A surgical navigation system (300), comprising
the system (100) of claim 14, wherein at least one of the fastening tool and the navigation pointer (310) are provided with one or more trackable optical markers; and
an optical sensor (320) capable of detecting light reflected or emitted by the one or more optical markers (330), wherein the surgical navigation system is configured to generate a first sensor signal indicative of the detected light.

16. The surgical navigation system (300) of claim 15, further comprising
a navigation controller (350) capable of receiving the first sensor signal, generating first sensor signal data based on the received first sensor signal and tracking at least one of the fastening tool and the navigation pointer (310) based on the generated first sensor signal data.

17. The surgical navigation system (300) of claim 15 or 16, further comprising at least one of
a medical imaging system (340) configured to generate medical imaging data of the patient's bone anatomy with the fiducial screw (106, 206) being inserted therein, and the medical imaging data.

18. The surgical navigation system (300) of claim 17, wherein
the navigation controller (350) is configured to register in a coordinate system the medical imaging data of the patient's bone anatomy, with the fiducial screw (106, 206) inserted therein, based at least on the first sensor signal data.

19. The surgical navigation system (300) of claim 17 or 18, wherein
the navigation controller (350) is configured to verify whether the medical imaging data of the patient's bone anatomy, with the fiducial screw inserted therein, are correctly registered in a, or the, coordinate system based at least on second sensor signal data generated based on a second sensor signal received from the optical sensor (320).
